(19) Europäisches Patentamt
European Patent Office
Office européen des brevets

(11) EP 1 533 296 A1

(12) **EUROPEAN PATENT APPLICATION**
published in accordance with Art. 158(3) EPC

(43) Date of publication:
**25.05.2005 Bulletin 2005/21**

(21) Application number: **03761759.4**

(22) Date of filing: **03.04.2003**

(51) Int Cl.[7]: **C07C 313/08**, C07B 59/00, G01N 33/68, G01N 27/62, A61K 38/00

(86) International application number:
**PCT/JP2003/004308**

(87) International publication number:
**WO 2004/002950 (08.01.2004 Gazette 2004/02)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **28.06.2002 JP 2002191496**
**28.06.2002 JP 2002191497**

(71) Applicant: **Shimadzu Corporation**
**Kyoto-shi, Kyoto 604-8511 (JP)**

(72) Inventors:
- **KUYAMA, Hiroki, c/o Shimadzu Corporation**
  **Kyoto-shi, Kyoto 604-8511 (JP)**
- **ANDO, Eiji, c/o Shimadzu Corporation**
  **Kyoto-shi, Kyoto 604-8511 (JP)**
- **NISHIMURA, Osamu, c/o Shimadzu Corporation**
  **Kyoto-shi, Kyoto 604-8511 (JP)**

(74) Representative: **Kilian, Helmut, Dr. et al**
**Wilhelms, Kilian & Partner**
**Patentanwälte**
**Eduard-Schmid-Strasse 2**
**81541 München (DE)**

# (54) SULFENYL COMPOUND, LABELING REAGENT, AND METHOD OF ANALYZING PEPTIDE

(57) A sulfenyl compound represented by the general formula: R-S-X (I)(wherein R represents an organic group having at least one constituent element labeled with an isotope, and X represents a leaving group); a labeling reagent comprising it; and a method of analyzing peptide using the labeling reagent. Preferably the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

Fig. 4

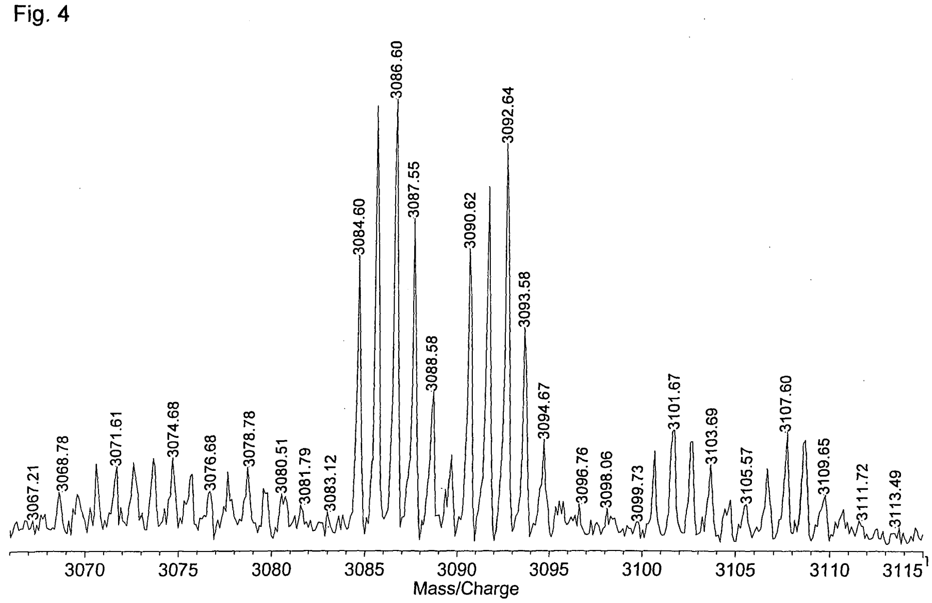


EP 1 533 296 A1

Printed by Jouve, 75001 PARIS (FR)

## Description

TECHNICAL FIELD

The present invention relates to exhaustive analysis of proteins expressed in living cells and tissues. More specifically, the present invention relates to compounds suitable for the analysis of expressed proteins, labeling reagents using such compounds, and quantitative analysis of expressed proteins using labeling agents.

BACKGROUND ART

Sulfenyl compounds are known to react with the indole ring of tryptophan residues and have been used as selective labeling reagents for tryptophan residues. Two-dimensional gel electrophoresis (2D-PAGE) is a common technique used in the analysis of proteins. In this technique, firstly proteins extracted from biological samples are separated/purified on 2D-PAGE and spots of interest are excised from the resulting two-dimensional gel image. The proteins in the spots are subsequently subjected to reductive alkylation, enzymatic digestion, and other processes and are then identified in a mass spectrometry (e.g., PMF). This technique also allows differential display of relative abundance of proteins in two biological samples.

This technique, however, has a drawback in that regulatory proteins and other proteins that are present in trace amounts can hardly be detected in the analysis of the lysates of whole cells in the case that the abundance of constitutively expressed proteins (housekeeping proteins) exists. The technique also has other drawbacks, including inability of differential display to accurately quantify proteins, non-reproducibility due to non-uniform gel plates, and difficulty in separating high molecular weight proteins.

With the arrival of post-genomic era, techniques have been developed to enable the mass spectrometry analysis of peptides. Among such techniques are techniques for efficient digestion of proteins, techniques for separation of complex polypeptide mixtures resulting from digestion of proteins, and ionization techniques used in mass spectrometry analysis of amino acid sequences of polypeptides. Now that these techniques are available, various analytical methods for peptides are proposed based on the principle of mass spectrometry and, as a result, a need has arisen for novel useful labeling reagents. One of the reagents developed to meet such a need is the isotope-coded affinity tag (ICAT). The technique using this reagent is one of the techniques for quantifying variation in the protein levels which was developed by Dr. Aebersold at the University of Washington (See, Non-patent article: R. Aebersold et al., Nature Biotech., 1999, 17, 994-999; and Patent Article: WO 00/11208). In the ICAT method, with aiming at cysteine residues of a protein, a specially designed alkylating reagent incorporating biotin is used to label SH groups of cysteine residues of a protein. This technique enables quantification of expressed biological proteins with significantly different abundance and, when used in conjunction with tandem mass spectrometry, allows identification of proteins whose expression levels varied.

However, it is expected that the above technique can result in complex mass spectra due to the cysteine residues present in proteins in large abundance. In addition, the ICAT method involves drawbacks that the use of a special avidin column to separate the labeled peptide fragments is needed and that the relatively large molecular weight of the reaction reagent (approx. 600) limits its reactivity.

DISCLOSURE OF THE INVENTION

Object of the Invention

Accordingly, it is an objective of the present invention to provide a sulfenyl compound that serves as a labeling reagent with superior reactivity and selectivity, can readily be analyzed by mass spectrometry, is highly quantitative, and does not require special columns. It is also an objective of the present invention to provide a labeling reagent using the sulfenyl compound, as well as an analytical method for proteins and peptides using such a labeling reagent.

Summary of the Invention

Over the course of our studies, the present inventors have found that these objectives can be achieved by using a sulfenyl compound having at least one constituent element labeled with an isotope. The finding ultimately led the present inventors to complete the present invention.

The present invention comprises following inventions.

(1) A sulfenyl compound represented by the general formula:

R-S-X (I)

(wherein R represents an organic group having at least one constituent element labeled with an isotope, and X represents a leaving group).

(2) The sulfenyl compound according to (1), wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

(3) The sulfenyl compound according to (1) or (2), wherein a molecular weight of the sulfenyl compound is larger than the molecular weight of a compound, which has the same structure as the compound and does not labeled with the isotope, by 3 to 12, preferably by 6 to 10.

(4) The sulfenyl compound according to any of (1) to (3), wherein the number of the constituent element labeled with the isotope is 3 or more, for example 3 to 12, preferably 6 to 10.

(5) The sulfenyl compound according to any of (1) to (4), wherein the organic functional group R is an alkyl group which may be substituted or an aryl group which may be substituted.

(6) The sulfenyl compound according to (5), wherein a substituent of the alkyl group which may be substituted is selected from the group consisting of $NO_2$, COOH, $SO_3H$, OH, alkoxyl, aryl, and aryloxy.

(7) The sulfenyl compound according to (5), wherein a substituent of the aryl group which may be substituted is selected from the group consisting of $NO_2$, COOH, $SO_3H$, OH, alkyl, alkoxyl, aryl, and aryloxy.

(8) The sulfenyl compound according to (5) or (7), wherein the organic group R is a phenyl group which may be substituted.

(9) The sulfenyl compound according to any of (1) to (8), wherein the leaving group X is a halogen atom.

(10) The sulfenyl compound according to any of (1) to (5) and (7) to (9), selected from the group consisting of 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

(11) A labeling reagent comprising a sulfenyl compound represented by the general formula:

R-S-X (I)

(wherein R represents an organic group having at least one constituent element labeled with an isotope, and X represents a leaving group).

(12) The labeling reagent according to (11), wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

(13) The labeling reagent according to (11) or (12), wherein a molecular weight of the sulfenyl compound is larger than the molecular weight of a compound, which has the same structure as the compound and does not labeled with the isotope, by 3 to 12, preferably by 6 to 10.

(14) The labeling reagent according to any of (1) to (13), wherein the number of the constituent element labeled with the isotope is 3 or more, for example 3 to 12, preferably 6 to 10.

(15) The labeling reagent according to any of (11) to (14), wherein the organic functional group R is an alkyl group which may be substituted or an aryl group which may be substituted.

(16) The labeling reagent according to (15), wherein a substituent of the alkyl group which may be substituted is selected from the group consisting of $NO_2$, COOH, $SO_3H$, OH, alkoxyl, aryl, and aryloxy.

(17) The labeling reagent according to (15), wherein a substituent of the aryl group which may be substituted is selected from the group consisting of $NO_2$, COOH, $SO_3H$, OH, alkyl, alkoxyl, aryl, and aryloxy.

(18) The labeling reagent according to (15) or (17), wherein the organic group R is a phenyl group which may be substituted.

(19) The labeling reagent according to any of (11) to (18), wherein the leaving group X is a halogen atom.

(20) The labeling reagent according to any of (11) to (15) and (17) to (19), wherein the sulfenyl compound is selected from the group consisting of 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

(21) The labeling reagent according to any of (11) to (20), in a use for peptide analysis.

Hereinafter, peptide is meant to encompass proteins in the present specification.

(22) The labeling reagent according to any of (11) to (21), separately including each of:

one compound (hereinafter, mentioned as "heavy reagent") selected from the sulfenyl compounds, and
a compound (hereinafter, mentioned as "light reagent") which has the same structure as the heavy reagent

and does not labeled with the isotope.

The labeling reagent of the present invention is preferably designed so that the difference in molecular weight between the heavy reagent and the light reagent is 3 to 12, and more preferably 6 to 10.

(23) A method of analyzing peptide, using a labeling reagent comprising a sulfenyl compound represented by the general formula:

$$R\text{-}S\text{-}X \qquad (I)$$

(wherein R represents an organic group having at least one constituent element labeled with an isotope, and X represents a leaving group).

(24) The method of analyzing peptide according to (23), wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

(25) The method of analyzing peptide according to (23) or (24), wherein a molecular weight of the sulfenyl compound is larger than the molecular weight of a compound, which has the same structure as the compound and does not labeled with the isotope, by 3 to 12, preferably by 6 to 10.

(26) The method of analyzing peptide according to any or (23) to (25), wherein the number of the constituent element labeled with the isotope is 3 or more, for example 3 to 12, preferably 6 to 10.

(27) The method of analyzing peptide according to (23) to (26), wherein the organic functional group R is an alkyl group which may be substituted or an aryl group which may be substituted.

(28) The method of analyzing peptide according to (27), wherein a substituent of the alkyl group which may be substituted is selected from the group consisting of $NO_2$, COOH, $SO_3H$, OH, alkoxyl, aryl, and aryloxy.

(29) The method of analyzing peptide according to (27), wherein a substituent of the aryl group which may be substituted is selected from the group consisting of $NO_2$, COOH, $SO_3H$, OH, alkyl, alkoxyl, aryl, and aryloxy.

(30) The method of analyzing peptide according to (27) or (29), wherein the organic group R is a phenyl group which may be substituted.

(31) The method of analyzing peptide according to any of (23) to (30), wherein the leaving group X is a halogen atom.

(32) The method of analyzing peptide according to any of (23) to (27) and (29) to (31), wherein the isotope-labeled sulfenyl compound of the formula (I) is selected from the group consisting of 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

(33) The method of analyzing peptide according to any of (23) to (32), wherein the labeling reagent separately including each of:

one compound selected from the sulfenyl compounds, and
a compound (light reagent) which has the same structure as the selected one compound (heavy reagent) and does not labeled with the isotope.

In the analysis of peptide of the present invention, the heavy reagent is preferably designed so that the difference in molecular weight between the heavy reagent and the light reagent is 3 to 12, and more preferably 6 to 10.

(34) The method of analyzing peptide according to any of (23) to (33), comprising labeling an amino acid residue of a peptide of interest by using the labeling reagent, and subjecting the resulting labeled peptide to mass spectrometry measurement.

(35) The method of analyzing peptide according to (34), comprising:

(i) labeling the peptide of interest with either one of the heavy reagent and the light reagent, to thereby obtain the labeled peptide of interest;
(ii) separately labeling a control peptide with the other of the heavy reagent and the light reagent to thereby obtain the labeled control peptide;
(iii) mixing the labeled peptide of interest obtained in (i) with the labeled controlled peptide obtained in (ii); and
(iv) subjecting the mixed labeled peptides to mass spectrometry measurement.

(36) The method of analyzing peptide according to (34) or (35), optionally comprising enzymatic digestion and/or a chemical treatment comprising reduction and alkylation.

(37) The method of analyzing peptide according to (36), wherein the enzymatic digestion is carried out before or

after the labeling.

(38) The method of analyzing peptide according to (36), wherein the chemical treatment is carried out before or after the labeling.

(39) The method of analyzing peptide according to (36), wherein the chemical treatment, the enzymatic digestion, and the labeling are carried out in this order.

(40) The method of analyzing peptide according to (36), wherein the chemical treatment, the labeling, and the enzymatic digestion are carried out in this order.

(41) The method of analyzing peptide according to (36), wherein the labeling, the chemical treatment, and the enzymatic digestion are carried out in this order.

(42) The method of analyzing peptide according to (34), comprising, after the labeling, optionally purifying the labeled peptide including separation by gel filtration and separation on a reversed-phase column.

(43) The method of analyzing peptide according to (34), wherein the amino acid residue is tryptophan residue.

(44) A novel compound comprising a peptide detected by method of analyzing peptide according to (23).

(45) A compound as a candidate for a potential pharmaceutical product comprising a peptide detected by the method of analyzing peptide according to (23).

BRIEF DESCRIPTION OF THE DRAWINGS

Fig. 1 is an LC chart of Example 3.

Fig. 2 is a graph showing the number of the pair peaks of labeled peptide fragments in separation on a column in Example 4.

Fig. 3 is a mass spectrum chart of Example 5.

Fig. 4 is a mass spectrum chart of Example 6.

Fig. 5 is a continuation of Fig. 4.

Fig. 6 is a mass spectrum chart of Example 8.

MODES FOR CARRYING OUT THE INVENTION

The present invention provides a sulfenyl compound represented by the following formula:

$$R\text{-}S\text{-}X \qquad (I)$$

where R is an organic functional group containing at least one constituent element labeled with an isotope, and X is a leaving group. This sulfenyl compound may be used as a labeling reagent to label a compound to be identified. The compound is particularly useful as a labeling reagent for peptide analysis.

A labeling reagent in which two individual reagents, one being the heavy reagent and the other the light reagent, are combined can facilitate mass spectrometry-based peptide analysis.

In the formula (I) above, the organic functional group R is a substituent group comprising C, H, and N, and optionally O and/or P as a constituent element. Preferably, the isotope is a stable isotope, such as $^{2}H$, $^{13}C$, $^{15}N$, $^{17}O$, and $^{18}O$.

When the labeling reagent in which the heavy reagent and the light reagent are combined is used, a peptide sample labeled with the heavy reagent is mixed with a peptide sample labeled with the light reagent and the mixture is subjected to mass spectrometry measurement. In this case, the presence of the isotope causes a difference in the mass number between the respective labeled peptides. This difference corresponds to the difference in molecular weight between the heavy reagent and the light reagent.

In this respect, the difference in mass number is preferably 3 to 12 daltons, and more preferably 6 to 10 daltons. The mass number difference that is 3 daltons or greater makes the tendency for the peak of the labeled peptide modified with the heavy reagent and the peak of the labeled peptide modified with the light reagent not to overlap, resulting in simple analysis.

To this end, the molecular weight of the sulfenyl compound of the present invention is preferably larger than the molecular weight of the compound having the same structure as the sulfenyl compound but unlabeled with the isotope by 3 to 12, and more preferably by 6 to 10. Thus, when the sulfenyl compound of the present invention contains only an isotope, such as $^{2}H$, $^{16}N$ or $^{17}O$, that has a greater mass number by 1, the number of the constituent elements labeled with isotopes is preferably 3 or more, for example, 3 to 12, and more preferably 6 to 10.

On the other hand, when the sulfenyl compound of the present invention contains an isotope, such as $^{18}O$,

that has a grater mass number greater by 2, 3 or more isotopes to achieve a mass number difference of 3 or larger may not be needed. For example, in the case of the sulfenyl compound containing only $^{18}O$ as the isotope, two of the constituent elements labeled with $^{18}O$ cause a difference of 4 daltons, resulting in giving a preferred mass difference.

When the labeleing reagent in which the sulfenyl compound of the present invention serves as the heavy reagent and used as labeling reagent combined with the light reagent, the molecular weight of the heavy reagent is designed so that the difference in molecular weight between the heavy reagent and the light reagent is preferably 3 to 12, and more preferably 6 to 10.

Preferably, the organic functional group R in the formula (I) is an alkyl group which may be substituted or an aryl group which may be substituted. Examples of the substituents of the alkyl group which may be substituted include, but are not limited to, $NO_2$, COOH, $SO_3H$, OH, alkoxyl, aryl, and aryloxy.

Examples of the substituents of the aryl group which may be substituted include, but are not limited to, $NO_2$, COOH, $SO_3H$, OH, alkyl, alkoxyl, aryl, and aryloxy.

These substituents may be selected to adjust the reactivity, solubility, and the like, of the present reagent.

Preferably, the organic functional group R is a phenyl group which may be substituted.

By having the properly designed organic functional group R, in the case of use as the labeling reagent, the labeled peptides can readily be separated on an ordinary column such as a reversed-phase column.

Examples of the leaving group X in the formula (I) include, but are not limited to, halogen atoms such as F, Cl, Br, and I.

The molecular weight of the sulfenyl compound of the present invention is preferably approximately 190 in terms of the reactivity with the peptides to be labeled.

Particularly preferred compounds for the sulfenyl compound of the present invention are 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

The sulfenyl compounds of the present invention may be synthesized from corresponding disulfides, thiols, or sulfides by halogenation or other techniques. For example, a manner for synthesizing a sulfenyl compound that has a phenyl group labeled with six $^{13}C$ atoms as the organic functional group R from, for example, a sulfide, is exemplified in the following manner.

First, the precursor sulfide is obtained as [$^{13}C_6$] phenyl benzyl sulfide by reacting [$^{13}C_6$] benzene derivative such as nitro[$^{13}C_6$]chlorobenzene with benzyl mercaptan.

Subsequently, the resulting [$^{13}C_6$] phenyl benzyl sulfide is reacted with a halogenating agent to obtain the desired sulfenyl compound. Sulfuryl chloride, bromine or the like may preferably be used as the halogenating agent. Tetrachloro carbon, ethylene chloride or the like may preferably be used as the solvent. The reaction is carried out for example at 10°C to 45°C for 5 min. to 1 hour.

Those skilled in the art may properly determine these conditions.

The sulfenyl compound thus obtained may be identified by comparing the mass spectrum of the obtained sulfenyl compound with that of the compound having the same structure as the sulfenyl compound but unlabeled with the isotope. The identification may also be performed by comparing $^{13}C$ and $^{1}H$ NMR spectra of the both compounds.

To employ the sulfenyl compound of the present invention can introduce the labeled substituent (R-S-group) into a compound to be identified resulting in the capability of use as a labeling reagent.

It is known that sulfenyl compounds can selectively react with the indole rings of tryptophan residues of a peptide. Therefore, the isotope-labeled sulfenyl compounds of the present invention are particularly useful as labeling reagents in the peptide analysis.

Tryptophan is an amino acid that plays a significant role in the aspects of the function and activity in proteins. Among tryptophan-containing peptides are adrenocorticotropic hormone (ACTH), Galanin, parathyroid hormone (PTH), α-chymotrypsin, gramicidin A, and lysozyme.

Tryptophan belongs to one of the least abundant amino acids in proteins and, thus, a peptide subjected to the labeling reaction and enzymatic digestion would give a simple mass spectrum. This facilitates analysis and offers a significant advantage to the proteome analysis.

Sulfenyl compounds (the isotope-labeled compounds of the present invention and the conventional, unlabeled compounds) undergo electrophilic substitution with the indole rings of the tryptophan residues, thereby labeling the tryptophan residues. This reaction is expected to have higher reactivity as compared to the nucleophilic substitution that takes place between the ICAT reagent and cysteine residues in the ICAT method.

The labeled tryptophan residue-including peptide which is generated by labeling by sulfenyl compound is more hydrophobic than the unlabeled tryptophan residue-including peptide and can thus be separated/purified without requiring a special column used in the ICAT method: These peptides can be separated/purified sufficiently on a gel filtration or reversed-phase column.

The method for analyzing peptides of the present invention involves labeling a peptide of interest at amino acid residues and subjecting the resulting labeled peptide to mass spectrometry measurement. The peptide of interest

may be a mixture of two or more peptides. The present invention comprises the steps of the labeling reaction and the mass spectrometry measurement. When a labeling reagent in which the heavy reagent and the light reagent are combined is used, the present invention comprises the steps of the labeling reaction, mixing the labeled peptides if necessary, and the mass spectrometry measurement.

If necessary, the present invention comprises the steps of fragmenting the peptide preferably by enzymatic treating (enzymatic digestion), and/or chemically treating by reduction and alkylation.

If necessary, the present invention further comprises the purification step comprising separation on a gel filtration column and separation on a reversed-phase column.

The process of the present invention, in which the respective steps of labeling, enzymatic digestion, and chemical treatment among the above process is combined, comprises: (a) process of carrying out enzymatic digestion followed by labeling; (b) process of carrying out labeling followed by enzymatic digestion; (c) process of carrying out chemical treatment followed by labeling; (d) process of carrying out labeling followed by chemical treatment; (e) process of carrying out chemical treatment, enzymatic digestion and labeling in this order; (f) process of carrying out chemical treatment, labeling and enzymatic digestion in this order; and (g) process of carrying out labeling, chemical treatment and enzymatic digestion in this order.

In using the labeling reagent in which the heavy reagent and the light reagent are combined, a control peptide sample is prepared in addition to the peptide sample of interest. When the peptide sample of interest is a mixture of two or more peptides, the control peptide sample may also be a mixture of corresponding two or more peptides. In one example of the sample, peptide sample of interest is proteins derived from diseased cells and the control peptide sample is proteins derived from normal cells corresponding to the diseased cells. In such a case, these corresponding samples are different from each other in peptide compositions. As the peptide sample of interest and the control peptide sample, respective samples may be used one kinds, and one sample and/or another sample may be used in combination with two or more kinds.

The labeling reaction is carried out by using an ordinary method. When the labeling reagent in which the heavy reagent and the light reagent are combined is used, the peptide of interest is labeled with using either one of the heavy reagent or the light reagent, and the other hand, the control peptide is labeled with using the other of the heavy reagent or the light reagent. Specifically, the peptide of interest may be labeled with using the heavy reagent and the control peptide may be labeled with using the light reagent; and the peptide of interest may be labeled with using the light reagent and the control peptide may be labeled with using the heavy reagent.

Such individual labeling gives at least two kinds of labeled peptides.

In the present invention, in addition to the labeling, peptides may be subjected to optional fragmentation. The fragmentation is preferably carried out by enzymatic digestion (hereinafter, the present invention will be described assuming that the fragmentation is carried out by enzymatic digestion). The enzymatic digestion may be carried out either prior to or following the above-described labeling process. Thus, the present invention comprises (a) process of carrying out enzymatic digestion followed by labeling, or (b) process of carrying out labeling followed by enzymatic digestion.

Preferred enzymes for the enzymatic digestion include trypsin, lysyl endopeptidase, thrombin, plasmin, kallikrein, and urokinase, each of which is an endopeptidase with high substrate specificity. Through the enzymatic digestion, peptides are fragmented into peptide fragments.

In the present invention, in addition to the labeling, an optional chemical treatment by reduction and alkylation. For the reasons described below, the chemical treatment and the labeling may be performed in any order. Specifically, the present invention comprises (c) process of carrying out chemical treatment followed by labeling, and (d) process of carrying out labeling followed by chemical treatment. Examples of the alkylation form include, but are not limited to, S-carbamidomethylation and S-carboxymethylation.

In some cases, a peptide of interest contains cysteine residues or disulfide bonds. The highly reactive SH groups of cysteine residues tend to form disulfide bonds and are converted to dithio groups, in some cases resulting in an unexpected peptide configuration. In a high pH environment, disulfide bonds tend to scramble with other disulfide bonds and, thus, the enzymatic digestion, normally carried out in a high pH environment, can give rise to conformational changes of peptides and makes analysis of the peptides difficult. This problem can be prevented by performing the chemical treatment. Specifically, in the chemical treatment, dithio groups which are susceptible to enzymatic digestion are reductively converted by the above-described reduction, and converted to alkylthio groups by the alkylation.

Accordingly, when the enzymatic digestion and the chemical treatment are carried out in combination (i.e, when labeling, enzymatic digestion, and enzymatic digestion are used in combination in the present invention), it is preferred that the chemical treatment precedes the enzymatic digestion.

As described, the enzymatic digestion may be carried out either prior to or following the labeling. Thus, when the enzymatic digestion (which follows the chemical treatment) precedes the labeling, the present invention comprises (e) process of carrying out chemical treatment, enzymatic digestion and labeling in this order.

On the other hand, in cases where the enzymatic digestion (which follows the chemical treatment) is carried

out subsequent to the labeling, the enzymatic digestion will be carried out in the last of the three steps. In this case, since the chemical treatment and the labeling precedes the enzymatic digestion may be carried out in any order, the present invention comprises (f) process of carrying out chemical treatment, labeling and enzymatic digestion in this order, or (g) process of carrying out labeling, chemical treatment and enzymatic digestion in this order.

The reason that the chemical treatment and the labeling may be carried out in any order is as follows:

When a peptide of interest contains cysteine residues, cysteine residues can be labeled, as can tryptophan residues, by the labeling of the present invention. Thus, in order to avoid this side reaction, or prevent the cysteine residues from being labeled, the chemical treatment may be performed prior to the labeling.

The chemical treatment may, however, be carried out following the labeling. As described above, when a peptide of interest contains cysteine residues, these cystaine residues bring about the side reaction during the labeling. At this time, cysteine residues react with the labeling reagent to form disulfide bonds. The disulfide bonds formed by the side reaction may then be subjected to the above-described chemical treatment for reductive cleavage and alkylation. Accordingly, the chemical treatment may be performed subsequent to the labeling in accordance with the present invention.

Thus, in the present invention the chemical treatment and the labeling may be carried out in any order.

In the manner described above, labeled peptides or labeled peptide fragments are obtained from a peptide of interest (labeled peptides and labeled peptide fragments are collectively referred to as "labeled peptide molecules," hereinafter. Further, peptides and peptide fragments which are unreacted labeling reaction are collectively referred to as "unlabeled peptide molecules," hereinafter). The labeled peptide molecules are preferably purified. The labeled peptide molecules, which are labeled at tryptophan residues, have higher hydrophobicity than the unlabeled peptide molecules, so that they can be readily separated from the unlabeled peptide molecules on an ordinary reversed-phase column using ODS or a similar material. Thus, a significant advantage of the present invention is that it does not require the use of special columns such as those used in the ICAT method.

Further, it is more preferred that the gel filtration is carried out prior to purification on a reversed-phase column. Examples of the gel filtration carriers include sephadex, biogel, and agarose gel, with sephadex particularly preferred. The most preferred sephadex is LH20. The gel filtration allows separation of unreacted or hydrolyzed labeling reagent and helps increase the purity of the labeled peptide molecules.

The optionally purified labeled peptide molecules are subjected to mass spectrometry measurement. When the labeling reagent in which the heavy reagent and the light reagent are combined is used, the peptide sample of interest and the control peptide sample may be mixed with each other prior to mass spectrometry measurement. In this manner, the labeled peptide molecules present in the respective samples are mixed with each other. The respective labeled peptide molecules may be mixed when they are subjected to mass spectrometry measurement, and the mixing may be preformed either upon enzymatic digestion, chemical treatment, or purification, provided that the mixing is performed following the labeling and prior to the mass spectrometry measurement. The mixed labeled peptide molecules are measured on mass spectrometry.

Examples of ionization techniques used in mass spectrometry in the present invention include FD, SIMS, FAB, MALDI, ESI, and the other methods. Examples of mass spectrometers for use in the present invention include double-focusing mass spectrometers, quadrupole mass spectrometers, Time-Of-Flight (TOF) mass spectrometers, Fourier transformation mass spectrometers, ion cyclotron mass spectrometers, and the other mass spectrometers.

According to the present invention, the results of mass spectrometry (MS) may be combined with the results of tandem mass spectrometry (MS/MS) for peptide analysis.

When the labeling reagent in which the heavy reagent and the light reagent are combined is used, the resulting mass spectrum (MS) will show pair peaks of fragment ions having a mass number difference that corresponds to the difference in molecular weight between the heavy reagent and the light reagent. Their integration ratio corresponds to the molar ratio of the labeled peptides present in the respective samples and, thus, to the molar ratio of the corresponding peptide of interest to the control peptide. In addition, the sequence information obtained from tandem mass spectrometry (MS/MS) and database search allows identification of the respective peptide of interest and the control peptide, corresponding to the respective labeled peptide molecules.

Some peptides present in either one of the peptide sample of interest or the control peptide sample may not be present in the other sample as their corresponding peptides. In other words, a peptide expressed in one of the samples may not be expressed in the other. In such a case, in the mass spectra, one signal will be found but the other corresponding signal will not be found. Thus, the signal for this peptide is difficult to find in the spectrum of the mixture of the samples. Therefore, in such a case, the entire course of the method for peptide analysis of the present invention, which comprises labeling, enzymatic digestion, chemical treatment, purification, and mass spectrometry measurement, may be performed individually for each sample. By comparing both spectra obtained so that the both spectra have the same scale on their horizontal axes (i.e., mass/charge (m/z)), and finding the signal whose relative abundance ratio is 1:0, the analysis may be performed.

As described above, the present invention can provide a labeling reagent that makes use of a particular

sulfenyl compound and thereby allows the labeling reaction in peptide analysis to be carried out at higher reactivity and selectivity. The use of such a highly reactive, highly selective labeling reagent in the peptide analysis enables more accurate and easier determination of the types of peptides as well as the difference in the expression levels of peptides which respectively exists in the sample of interest and the control sample. The use of this labeling reagent also enables more accurate and easier determination what abundance of a particular peptide contains in the sample compared to the other peptide in the sample. Thus, the present invention enables the exhaustive analysis of expressed proteins more quantitative and more efficient. The method of the present invention enables to determine the difference in the peptide composition between normal cells and diseased cells at the level of expressed proteins and, thus, provide a way to unveil underlying mechanisms that control activation of disease cells. Namely, in case that a peptide proven by the method of the present invention is involved in the onset of a particular disease, by theoretically designing compound which is targeted to the peptide and specifically act on the peptide to effectively control its function on computer, a candidate for a potential pharmaceutical product can be created. Also, information on the gene encoding this peptide is obtained, and a substance to specifically control expression of the gene can be created as a candidate for a potential pharmaceutical product. The present invention enables to create of such candidate compounds more effectively.

EXAMPLES

The present invention will now be described in further detail with reference to examples, which do not limit the scope of the present invention.

[Example 1: Synthesis of sulfenyl compound]

In this example, 2-nitro[$^{13}C_6$] benzenesulfenyl chloride (heavy reagent) was synthesized from a corresponding sulfide.

(Synthesis of sulfide)

1g (6.1x$10^{-3}$mol) of 2-nitrochloro[$^{13}C_6$] benzene was dissolved in 1ml methanol. To this, 0.8g (6.6x$10^{-3}$mol) benzyl mercaptan and 0.8ml (0.01mol) pyridine were added and refluxing was performed for 16 hours while heated. By cooling to room temperature, 2-nitro[$^{13}C_6$]phenyl benzyl sulfide was crystallized. The formed crystal was collected by filtration and was washed with methanol, and drying with air. This gave 1.38g (90%) of 2-nitro[$^{13}C_{6]}$phenyl benzyl sulfide as a yellow crystal.

(Synthesis of heavy reagent)

To 1.38g (5.5x$10^{-3}$mol) of 2-nitro[$^{13}C_6$]phenyl benzyl sulfide obtained above, 10ml ethylene chloride and then 940mg (7.9x$10^{-3}$mol) sulfuryl chloride were added, and stirring was performed at room temperature. The reaction mixture was concentrated under reduced pressure while maintained at 50 to 60°C in a heat bath. A petroleum ether was added to the resultant oily product and the resulting crystal was collected by filtration. The crystal was washed with a petroleum ether and was air-dried to give 938g (87%) of 2-nitro[$^{13}C_6$] benzenesulfenyl chloride.

(Identification)

The resulting heavy reagent and the corresponding light reagent (2-nitro[$^{12}C_6$] benzenesulfenyl chloride) were each subjected to mass spectrometry measurement. The structural formula of the heavy reagent and the light reagent are shown in the following chemical formula 1:

Chemical formula 1

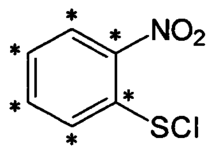

heavy reagent

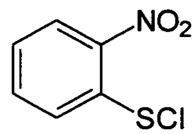

light reagent

where * indicates a carbon molecule labeled with $^{13}C$.

heavy reagent

(m/z) 195.10($M^+$, 34.1), 160.10(15.2), 144.10(4.7), 131.10(25.0), 114.10(15.6), 99.20(100.0), 83.10(10.6), 71.20(32.3), 54.20(12.3)

light reagent

(m/z) 189.00($M^+$, 35.3), 154.10(15.8), 138.10(4.9), 125.10(26.3), 108.10(17.6), 93.10(100.0), 78.10(14.1), 66.10(32.2), 50.20(15.3)

Comparison of the mass spectra of the heavy reagent and the light reagent revealed a difference of 6 daltons for all of the molecular ions and the fragment ions having a benzene ring. Each of the fragment peaks was substantially the same intensity.

[Example 2: Separation experiment]

Using ACTH, Galanin, and PTH as model peptides and 2-nitro[$^{12}C_6$] benzenesulfenyl chloride (light reagent) as the labeling reagent, an experiment was conducted to see if the labeled peptides can be separated from their unlabeled peptides.

160µg ($5.4x10^{-8}$mol) ACTH was added to 100µl of a 70% aqueous solution of acetic acid. To this, 0.41mg (40mol) of 2-nitro[$^{12}C_6$] benzenesulfenyl chloride was added and stirring was performed by vortex mixer at room temperature for 1 hour. The reaction mixture was chilled in an ice bath, followed by addition of 800µl ice-chilled ether and centrifugation. The resulting precipitate was washed with ice-chilled ether to obtain the labeled form. The labeled peptide was mixed with the peptide not subjected to labeling and separation was performed on a reversed-phase column (LC). The same procedures were followed for Galanin and PTH, and the respective retention time was showed as follows. The results are as shown below (labeled peptides are indicated by "mod-."). The enzymatic digestion was omitted because of the simplified system.

(Retention time)

| | |
|---|---|
| ACTH | 16.57min. |
| mod-ACTH | 34.78min. |
| Galanin | 21.78min. |
| mod-Galanin | 35.27min. |
| PTH | 24.78min. |
| mod-PTH | 35.47min. |

(Conditions)

| | |
|---|---|
| Buffer A | 0. 01M HCOOH-$Et_3N$ (pH4.5) |
| Solution B | $CH_3CN$ |
| Gradient of solution B | varied from 0% to 100% over a 50 minute time period. |

(continued)

| | |
|---|---|
| Column | Shimpack VP-ODS, 250x4.5mm |

As shown above, between the labeled form and the unlabeled form, a 10 to 18 minute difference was observed in the retention time for all of the three types. This demonstrates that only labeled peptide can be separated by LC.

Further, using 2-nitro[$^{13}C_6$] benzenesulfenyl chloride (heavy reagent), the same separation experiment was conducted and results in the same retention time. This indicates that using the heavy reagent also enables that only labeled peptide is separated.

[Example 3: Separation experiment]

(Labeling)

Using lysozyme as a model protein, an experiment was conducted for the same purpose as in Example 2. In this experiment, the labeling, chemical treatment, and enzymatic digestion were performed in this order.

Lysozyme was labeled with the "light reagent" in the same manner as in Experiment 2, and then washed and freeze-dried. Subsequently, the chemical treatment (reduction of disulfide linkages and S-carbamidomethylation) and the enzymatic digestion, as described below, was performed.

(Reduction of disulfide bonds)

100mM $NH_4HCO_3$ was added to this for dissolving, 50 molar equivalents of DTT with respect to one disulfide bond were added, and the reaction was allowed to proceed at 56°C for 1 hour.

(S-carbamidomethylation)

After the reaction mixture was allowed to cool to room temperature, 100mM $NH_4HCO_3$ solution of iodoacetamide was added and the reaction was allowed to proceed at room temperature for 1 hour. The reaction solution was subjected to purification on a Sephadex G10 column and was freeze-dried.

(Enzymatic digestion)

Trypsin in a 50mM $NH_4HCO_3$-5mM $CaCl_2$ solution was added to this in a proper amount and the reaction was allowed to proceed at 37°C for 16 hours.

Subsequently, the digest obtained by the above series of treatments and containing the labeled peptide fragments was subjected to separation by LC. The chart of LC is shown in Fig. 1, in which horizontal axis represents the retention time (min) and vertical axis represents the peak intensity (mAU). As a result, all of the desired labeled peptide fragments were contained in the fraction eluted at 29 to 39min (detected fraction a). Table 1 shows a comparison between the mass/charge ratios (m/z) in mass spectrometry of the lysozyme-derived peptides before LC and lysozyme-derived peptide fragments observed in the detected fraction a after LC. In Table 1, the figures in parentheses indicate the numbers in the amino acid sequence and the figures assigned "W" indicate the peptide fragments containing labeled tryptophan.

Table 1

| before LC | after LC (Fr.a) |
|---|---|
| 874.81 (15-21) | |
| 1198.86 (117-125W) | 1198.45 (117-125W) |
| 1299.74 (62-68W) | 1299.25 (62-68W) |
| 1429.06 (34-45) | |
| 1479.03 (22-33W) | 1479.38 (22-33W) |
| 1487.07 (115-125W) | 1486.42 (115-125W) |
| 1754.34 (46-61) | |

(Conditions)

| | |
|---|---|
| Buffer A | 0. 1% TFA |
| Solution B | 0.07 TFA in $CH_3CN$ |
| Gradient of solution B | varied from 0% to 100% over a 100 minute time period. |
| Column | Shimpack VP-ODS, 150x4.6mm |

The results shown above demonstrate that labeled peptides can be separated by LC.
Further, using 2-nitro[$^{13}C_6$] benzenesulfenyl chloride (heavy reagent), the same separation experiment was conducted, and it is found that using the heavy reagent also enables that only labeled peptide is separated.

[Example 4: Separation experiment]

In this example, an experiment was conducted using rat serum (Wister) to see how the desired tryptophan-containing peptide fragments are actually concentrated over the course of the labeling, enzymatic digestion, and separation/purification. Two 2µl samples of the rat serum (100µg of total amount of proteins) were prepared. In the same manner as in Example 2, one sample was labeled with the "light reagent" and the other with the "heavy reagent", and these were mixed with each other. In the same manner as Example 3, the chemical treatment and enzymatic digestion were performed. This gave a digest containing labeled peptide fragments. Subsequently, the separation/purification was carried out using Sephadex LH20 column in the following conditions:

| | |
|---|---|
| Column volume | 2.0ml |
| Fraction volume | 120µl |
| Eluant | 30% acetonitrile |

Following the fractionation, each fraction was subjected to MALDI-TOF MS and the pair peaks of labeled peptide fragments that are 6 daltons apart were observed. Fig. 2 is a histogram showing counted number of the pair peaks. In Fig. 2, horizontal axis represents the fraction number and vertical axis represents the number of pair peaks. As shown by Fig. 2, desired pair peaks were observed from fractions No. 16 to No. 23. Namely, it was found that a fraction may be collected in total 1ml from 1.8ml to 2.8ml, and may actually be collected in a somewhat larger volume.

[Example 5]

In this example, a peptide containing both tryptophan and cysteine residues was used. As in Examples 3 and 4, not only tryptophan residues but also cysteine residues are labeled when the peptide is labeled prior to the chemical treatment, so that the peptide labeled both on cysteine residues and on tryptophan residues can be generated immediately after the labeling. An experiment was conducted to determine if the peptide is converted to the peptide labeled only on the tryptophan residues after the chemical treatment.
Presinilin-1 (331-349)-Cys, a peptide having a sequence of NDDGGFSEEWEAQRDSHLGC (SEQ ID NO: 1) (Bachem, MW = 2252.3) was used, and labeling was performed in the same manner as in Example 2. The MALDI-TOF MS spectrum chart for the peptide before labeling (signal intensity = 108mV) is shown at the bottom of Fig. 3, and the MALDI-TOF MS spectrum chart for the labeled peptide (signal intensity = 21mV) is shown in the middle of Fig. 3. In all of the charts in Fig. 3, horizontal axis represents the mass/charge ratio (m/z) and vertical axis represents the relative ion intensity (%Int.). As shown in these charts, the peptide of SEQ ID NO: 1 that gave a signal at 2252.04 (m/z) was converted to a compound that gave a signal at 2559.44 (m/z). This compound corresponds to a peptide having a sequence of NDDGGFSEEW(Nbs)EAQRDSHLGC(Nbs) (SEQ ID NO: 2), which is labeled on both tryptophan and cysteine. In the sequence, W(Nbs) represents a labeled tryptophan residue and C(Nbs) represents a labeled cysteine residue.
A series of chemical treatment comprising reduction and alkylation was performed respectively using TCEP and iodoacetamide. The MALDI-TOF MS spectrum chart of the resulting peptide (signal intensity = 60mV) is shown at the top of Fig. 3. As can be seen from these spectra, the peptide of SEQ ID NO: 2 that gave a signal at 2559.44 (m/z) was converted to a compound that gave a signal at 2436.6 (m/z). This compound is a peptide having a sequence of NDDGGFSEEW(Nbs)EAQRDSHLGC(AM) (SEQ ID NO: 3), which has the labeled substituent on the cysteine residue due to labeling cleaved by the reducing agent and then amidomethylated (AM) by iodoacetamide. In the sequence,

C(AM) represents an amidomethylated cysteine residue.

[Example 6: Quantitative analysis]

First, using ACTH and Galanin as sample peptides, a model of cells in two different conditions was established by setting the ratio of the peptides contents as follows:

| | |
|---|---|
| Cells A ACTH | Galanin = 1:1 (molar ratio) |
| Cells B ACTH | Galanin = 1:2 (molar ratio) |
| The ratio of ACTH content in cells A to that in cells B Cells A | Cells B = 1:1 (molar ratio) |
| The ratio of Galanin content in cells A to that in cells B Cells A | Cells B = 1:2 (molar ratio) |

Second, in each system, the labeling reaction was performed in the same manner as in Example 2 using the "light reagent" for the cells A and the "heavy reagent" for the cells B. The chemical treatment and the enzymatic digestion were omitted because of the simplified systems.
Further, following the labeling reaction, the two systems were mixed with each other and subjected to gel filtration on Sephadex G25.
The peptides obtained through the gel filtration were subjected to LC so that only labeled peptide was separated. The resulting labeled peptides were subjected to MALDI-TOF MS measurement. The resulting mass spectrum was shown in Fig. 4 and Fig. 5 (continuation of Fig. 4). In Figs. 4 and 5, horizontal axis represents the mass/charge ratio (m/z) and vertical axis represents the relative ion intensity (%Int.).
In the results of the mass spectrometry, the predetermined ratios of the peptides are accurately reflected by the peak intensity ratios of the respective ions. The peptides modified with the "light reagent" are denoted by L-mod and those modified with the "heavy reagent" are denoted by H-mod.
Ion peaks
(m/z) = 3086.60 [ACTH(L-mod)], 3092.64 [ACTH(H-mod)], 3316.68 [Galanin (L-mod)], 3322.68 [Galanin (H-mod)]
Peak intensity ratios
ACTH (L-mod): ACTH (H-mod) = 1:1
Galanin (L-mod): Galanin (H-mod) = 1:2

[Example 7: Quantitative analysis]

Using α-lactoalbumin (LCA_BOVIN), glycelaldehyde-3-phosphate dehydrogenase (G3P_RABIT), phosphorylase B (PHS2_RABIT), and ovalbumin (OVAL_CHICK) (each available from Sigma) as model peptides, a model of cells in two different conditions was established by setting the ratio of the peptides contents as follows:

| | |
|---|---|
| Cells A | LCA_BOVIN, G3P_RABIT, PHS2_RABIT, OVAL_CHICK (12.5μg of each was contained) |
| Cells B | LCA_BOVIN, G3P_RABIT, PHS2_RABIT, OVAL_CHICK (25μg of each was contained) |
| The ratio of peptide content in cells A to that in cells B | Cells A: Cells B = 1:2 (molar ratio) |

In each system, the labeling reaction was then performed in the same manner as in Example 2 using the "light reagent" for the cells A and the "heavy reagent" for the cells B, followed by the chemical treatment and the enzymatic digestion performed in the same manner as Example 3. This gave a digest containing labeled peptide fragments. The digest was then subjected to separation/purification on a Sephadex LH20 column and then to MALDI-TOF MS analysis. The results are shown in Table 2. Shown in Table 2 are gene names; identified peptide sequences (Peptide Sequence Identified); observed ratios of the amount of peptide labeled with the light reagent to the amount of peptide labeled with the heavy reagent (Observed Ratio ($^{13}C_0/^{13}C_6$)); expected ratios of the amount of peptide labeled with the light reagent to the amount of peptide labeled with the heavy reagent (Expected Ratio ($^{13}C_0/^{13}C_6$)); differences between the mean values of the observed values and the expected ratios (Means+-SD); and errors (Error (%)).
As shown in Table 2, tryptophan-containing peptide fragments derived from the labeled peptide fragments were identified for each of the sample peptides used. In addition, each of the observed ratios had a 9% or less error, showing a good consistency with the corresponding expected value.

Table 2

| Gene Name | Peptide Sequence Identified | Observed Ratio ($^{13}C_0$ / $^{13}C_6$) | Expected Ratio ($^{13}C_0$ / $^{13}C_6$) | Mean+-SD | Error (%) |
|---|---|---|---|---|---|
| LCA_BOVIN | IWCK | 0.48 | 0.5 | 0.46+-0.04 | 4 |
| | LDQWLCEK | 0.43 | | | |
| | LDQWLCEKL | 0.51 | | | |
| | VGINYNLAHK | 0.40 | | | |
| OVAL_CHICK | GLWEK | 0.45 | 0.5 | 0.54+-0.09 | 9 |
| | GLWEKAFK | 0.63 | | | |
| G3P_RABIT | LWR | 0.48 | 0.5 | 0.49+-0.01 | 1 |
| | LISWYDNEFGYSNR | 0.50 | | | |
| PHS2_RABIT | WIR | 0.48 | 0.5 | 0.45+-0.03 | 2 |
| | EWTR | 0.46 | | | |
| | EWTRMVIR | 0.42 | | | |

(identified peptide fragment)

LCA_BOVIN-derived
IWCK (SEQ ID NO: 4)
LDQWLCEK (SEQ ID NO: 5)
LDQWLCEKL (SEQ ID NO: 6)
VGINYNLAHK (SEQ ID NO: 7)
G3P_RABIT-derived
GLWEK (SEQ ID NO: 8)
GLWEKAFK (SEQ ID NO: 9)
PHS2_RABIT-derived
LWR
LISWYDNEFGYSNR (SEQ ID NO: 10)
OVAL_CHICK-derived
WIR
EWTR (SEQ ID NO: 11)
EWTRMVIR (SEQ ID NO: 12)

[Example 8: Quantitative analysis]

In this example, egg-white lysozyme (Sigma) to serve as a sample protein was added to rat serum (Wister) to determine if any signals derived from lysozyme can be observed (Spike test). A model of cells in two different conditions was established by setting the amounts of the peptides as follows:

| | |
|---|---|
| Cells A | 5μl rat serum + 3μg (0.21nmol) egg-white lysozyme |
| Cells B | 5μl rat serum + 6μg (0.42nmol) egg-white lysozyme |
| The ratio of lysozyme content in cells A to that in cells B | Cells A: Cells B = 1:2 (molar ratio) |

The total amount of proteins and the amount of albumin present in 5μl of the rat serum were 0.28mg and 0.14mg (2.1nmol), respectively.

In each type of the cells, the labeling reaction was then performed in the same manner as in Example 2 using the "heavy reagent" for the cells A and the "light reagent" for the cells B. Following the labeling reaction, the two solutions were mixed with each other and the chemical treatment and enzymatic digestion was performed in the same manner as in Example 3. This gave a digest containing labeled peptide fragments. The digest was subjected to separation/purification on a Sephadex LH20 column and then to MALDI-TOF MS analysis. The resulting spectrum chart is shown in Fig. 6. In Fig. 6, horizontal axis represents the mass/charge ratio (m/z) and vertical axis represents the relative ion intensity (%Int.). As shown in Fig. 6, lysozyme-derived fragments were detected as peaks at 1199.33 and 1205.27 (m/z) with the peak intensity ratio of 2:1. Thus, the observed ratio (*i.e.*, 2) was consistent with the expected ratio (*i.e.*, 2) with an error of 0%. This indicates that also in biological samples, the relative abundance of the added lysozymes was directly reflected as the relative abundance of the peptide fragments obtained after enzymatic digestion.

As demonstrated by the results of the above-described examples, by using the method of the present invention, all of the errors is 9% or less and is thus highly quantitative.

[Example 9: Quantitative analysis]

In this example, a quantitative analysis was performed using actual biological samples.

Rat serums of the following two lines of rats were used.

A: SPF/VAF Crj Wister rats (normal rats)
B: SPF/VAF GK/Crj rats (hyperglycemic rats, blood glucose level = 372mg/dl)

2μl serum from a rat of each line was used as a sample. Each sample was quantitatively analyzed in the same manner as in Example 7, except that the samples were subjected to ESI-MS/MS analysis using LCQ DecaXP (Thermo Finnigan)-LCAd-Vp system (Shimadzu Corporation). The results are shown in Table 3 and include gene names (Gene Name), identified peptide sequences (Peptide Sequence Identified), and observed ratios of the amounts of normal rat peptides to the amounts of hyperglycemic rat peptides (Observed Ratio (Wister/GK)).

Table 3

| Gene Name | Peptide Sequence Identified | Observed Ratio (Wister / GK) |
|---|---|---|
| Alb | AWAVAR | 1 |
| transferrin | NTAAWAK | 1 |
| P2rx7 | WKIRK | 1 |

(identified peptide fragment)

AWAVAR (SEQ ID NO: 13)
NTAAWAK (SEQ ID NO: 14)
WKIRK (SEQ ID NO: 15)

In each of Examples above, 2-nitro[$^{13}C_6$] benzenesulfenyl chloride was synthesized as the sulfenyl compound and was used as a labeling reagent to analysis of each of ACTH, Galanin, PTH, lysozyme, rat serum, presinilin-1 (331-349)-Cys, α-lactoalbumin, glycelaldehyde-3-phosphate dehydrogenase, phosphorylase B, and ovalbumin. The present invention, however, is not limited to the above-described sulfenyl compound, but encompasses any sulfenyl compound represented by the general formula (I). Also, the labeling reagent of the present invention may be any sulfenyl compound represented by the general formula (I). Further, the method of the present invention is not limited to the above-described peptides, but encompasses any peptide including proteins. Therefore, the foregoing description of Examples is intended to be only illustrative and should not be construed in any way as limiting the scope of the invention. The invention is intended to cover all changes, modifications and equivalents that may be included within the spirit and the scope of the invention as defined by the appended claims.

In the free text of sequence listing (i.e., Description of Artificial Sequence), SEQ ID NO: 1 represents Presenilin-1 (331-349)-Cys, SEQ ID NO: 2 represents labeled Presenilin-1 (331-349)-Cys, and SEQ ID NO: 3 represents labeled and amidomethylated Presenilin-1 (331-349)-Cys.

INDUSTRIAL APPLICABILITY

The present invention provides a sulfenyl compound that serves as a labeling reagent with superior reactivity and selectivity, can readily be analyzed by mass spectrometry, is highly quantitative, and does not require special columns. The present invention also provides a labeling reagent using the sulfenyl compound, as well as an analytical

method for proteins and peptides using such a labeling reagent.

## Claims

**1.** A sulfenyl compound represented by the general formula:

$$R\text{-}S\text{-}X \qquad (I)$$

(wherein R represents an organic group having at least one constituent element labeled with an isotope, and X represents a leaving group).

**2.** The sulfenyl compound according to claim 1, wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

**3.** The sulfenyl compound according to claim 1, wherein a molecular weight of the sulfenyl compound is larger than the molecular weight of a compound, which has the same structure as the compound and does not labeled with the isotope, by 3 to 12.

**4.** The sulfenyl compound according to claim 1, wherein the number of the constituent element labeled with the isotope is 3 or more.

**5.** The sulfenyl compound according to claim 1, wherein the organic functional group R is an alkyl group which may be substituted or an aryl group which may be substituted.

**6.** The sulfenyl compound according to claim 5, wherein a substituent of the alkyl group which may be substituted is selected from the group consisting of $NO_2$, COOH, $SO_3H$, OH, alkoxyl, aryl, and aryloxy.

**7.** The sulfenyl compound according to claim 5, wherein a substituent of the aryl group which may be substituted is selected from the group consisting of $NO_2$, COOH, $SO_3H$, OH, alkyl, alkoxyl, aryl, and aryloxy.

**8.** The sulfenyl compound according to claim 5, wherein the organic group R is a phenyl group which may be substituted.

**9.** The sulfenyl compound according to claim 1, wherein the leaving group X is a halogen atom.

**10.** The sulfenyl compound according to claim 1, selected from the group consisting of 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

**11.** A labeling reagent comprising a sulfenyl compound represented by the general formula:

$$R\text{-}S\text{-}X \qquad (I)$$

(wherein R represents an organic group having at least one constituent element labeled with an isotope, and X represents a leaving group).

**12.** The labeling reagent according to claim 11, wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

**13.** The labeling reagent according to claim 11, wherein a molecular weight of the sulfenyl compound is larger than the molecular weight of a compound, which has the same structure as the compound and does not labeled with the isotope, by 3 to 12.

**14.** The labeling reagent according to claim 11, wherein the number of the constituent element labeled with the isotope is 3 or more.

**15.** The labeling reagent according to claim 11, wherein the organic functional group R is an alkyl group which may be substituted or an aryl group which may be substituted.

**16.** The labeling reagent according to claim 15, wherein a substituent of the alkyl group which may be substituted is selected from the group consisting of $NO_2$, COOH, $SO_3H$, OH, alkoxyl, aryl, and aryloxy.

**17.** The labeling reagent according to claim 15, wherein a substituent of the aryl group which may be substituted is selected from the group consisting of $NO_2$, COOH, $SO_3H$, OH, alkyl, alkoxyl, aryl, and aryloxy.

**18.** The labeling reagent according to claim 15, wherein the organic group R is a phenyl group which may be substituted.

**19.** The labeling reagent according to claim 11, wherein the leaving group X is a halogen atom.

**20.** The labeling reagent according to claim 11, wherein the sulfenyl compound is selected from the group consisting of 2-nitro[$^{13}C_6$] benzenesulfenyl chloride, 4-nitro [$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

**21.** The labeling reagent according to claim 11, in a use for peptide analysis.

**22.** The labeling reagent according to claim 11, separately including each of:

one compound selected from the sulfenyl compounds, and
a compound (light reagent) which has the same structure as the selected one compound (heavy reagent) and does not labeled with the isotope.

**23.** A method of analyzing peptide, using a labeling reagent comprising a sulfenyl compound represented by the general formula:

$$R\text{-}S\text{-}X \qquad (I)$$

(wherein R represents an organic group having at least one constituent element labeled with an isotope, and X represents a leaving group).

**24.** The method of analyzing peptide according to claim 23, wherein the organic group R comprises C, H, and N, and optionally O and/or P as the constituent element, and the isotope is a stable isotope selected from the group consisting of $^{2}H$, $^{13}C$, $^{15}N$, $^{17}O$, and $^{18}O$.

**25.** The method of analyzing peptide according to claim 23, wherein a molecular weight of the sulfenyl compound is larger than the molecular weight of a compound, which has the same structure as the compound and does not labeled with the isotope, by 3 to 12.

**26.** The method of analyzing peptide according to claim 23, wherein the number of the constituent element labeled with the isotope is 3 or more.

**27.** The method of analyzing peptide according to claim 23, wherein the organic functional group R is an alkyl group which may be substituted or an aryl group which may be substituted.

**28.** The method of analyzing peptide according to claim 27, wherein a substituent of the alkyl group which may be substituted is selected from the group consisting of $NO_2$, COOH, $SO_3H$, OH, alkoxyl, aryl, and aryloxy.

**29.** The method of analyzing peptide according to claim 27, wherein a substituent of the aryl group which may be substituted is selected from the group consisting of $NO_2$, COOH, $SO_3H$, OH, alkyl, alkoxyl, aryl, and aryloxy.

**30.** The method of analyzing peptide according to claim 27, wherein the organic group R is a phenyl group which may be substituted.

**31.** The method of analyzing peptide according to claim 23, wherein the leaving group X is a halogen atom.

**32.** The method of analyzing peptide according to claim 23, wherein the isotope-labeled sulfenyl compound of the formula (I) is selected from the group consisting of 2-nitro[$^{13}C_6$] benzenesulfenyl chloride, 4-nitro[$^{13}C_6$] benzenesulfenyl chloride, 2, 4-dinitro[$^{13}C_6$] benzenesulfenyl chloride, and 2-nitro-4-carboxy[$^{13}C_6$] benzenesulfenyl chloride.

**33.** The method of analyzing peptide according to claim 23, wherein the labeling reagent separately including each of:

one compound selected from the sulfenyl compounds, and
a compound (light reagent) which has the same structure as the selected one compound (heavy reagent) and does not labeled with the isotope.

**34.** The method of analyzing peptide according to claim 23, comprising labeling an amino acid residue of a peptide of interest by using the labeling reagent, and subjecting the resulting labeled peptide to mass spectrometry measurement.

**35.** The method of analyzing peptide according to claim 34, comprising:

(i) labeling the peptide of interest with either one of: one compound (heavy reagent) selected from the sulfenyl compounds; and a compound (light reagent) which has the same structure as the selected one compound and does not labeled with the isotope, to thereby obtain the labeled peptide of interest;
(ii) separately labeling a control peptide with the other of the heavy reagent and the light reagent to thereby obtain the labeled control peptide;
(iii) mixing the labeled peptide of interest obtained in (i) with the labeled controlled peptide obtained in (ii); and
(iv) subjecting the mixed labeled peptides to mass spectrometry measurement.

**36.** The method of analyzing peptide according to claim 34, optionally comprising enzymatic digestion and/or a chemical treatment comprising reduction and alkylation.

**37.** The method of analyzing peptide according to claim 36, wherein the enzymatic digestion is carried out before or after the labeling.

**38.** The method of analyzing peptide according to claim 36, wherein the chemical treatment is carried out before or after the labeling.

**39.** The method of analyzing peptide according to claim 36, wherein the chemical treatment, the enzymatic digestion, and the labeling are carried out in this order.

**40.** The method of analyzing peptide according to claim 36, wherein the chemical treatment, the labeling, and the enzymatic digestion are carried out in this order.

**41.** The method of analyzing peptide according to claim 36, wherein the labeling, the chemical treatment, and the enzymatic digestion are carried out in this order.

**42.** The method of analyzing peptide according to claim 34, comprising, after the labeling, optionally purifying the labeled peptide including separation by gel filtration and separation on a reversed-phase column.

**43.** The method of analyzing peptide according to claim 34, wherein the amino acid residue is tryptophan residue.

**44.** A novel compound comprising a peptide detected by the method of analyzing peptide according to claim 23.

**45.** A compound as a candidate for a potential pharmaceutical product comprising a peptide detected by the method of analyzing peptide according to claim 23.

Fig. 1

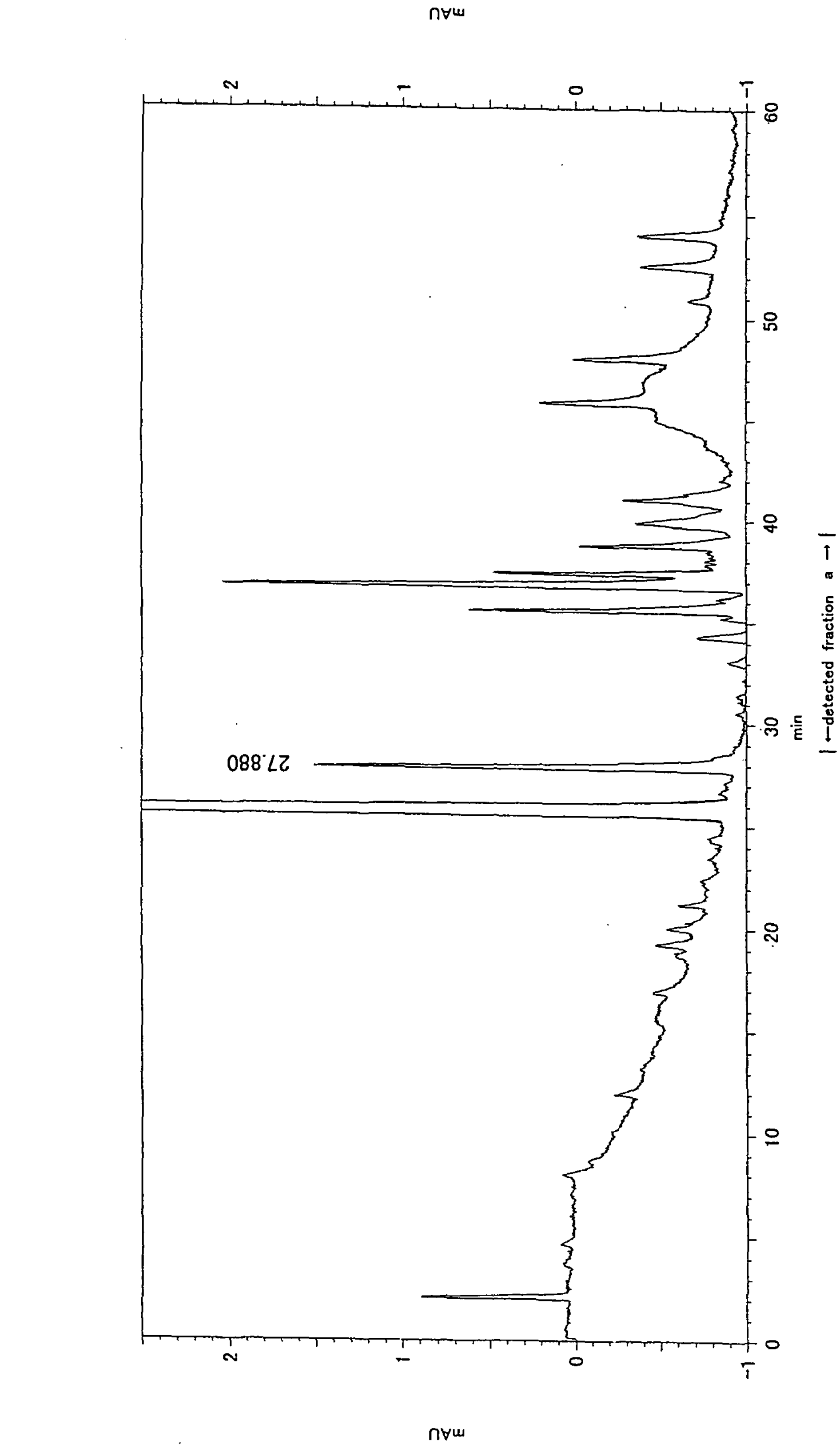

Fig. 2

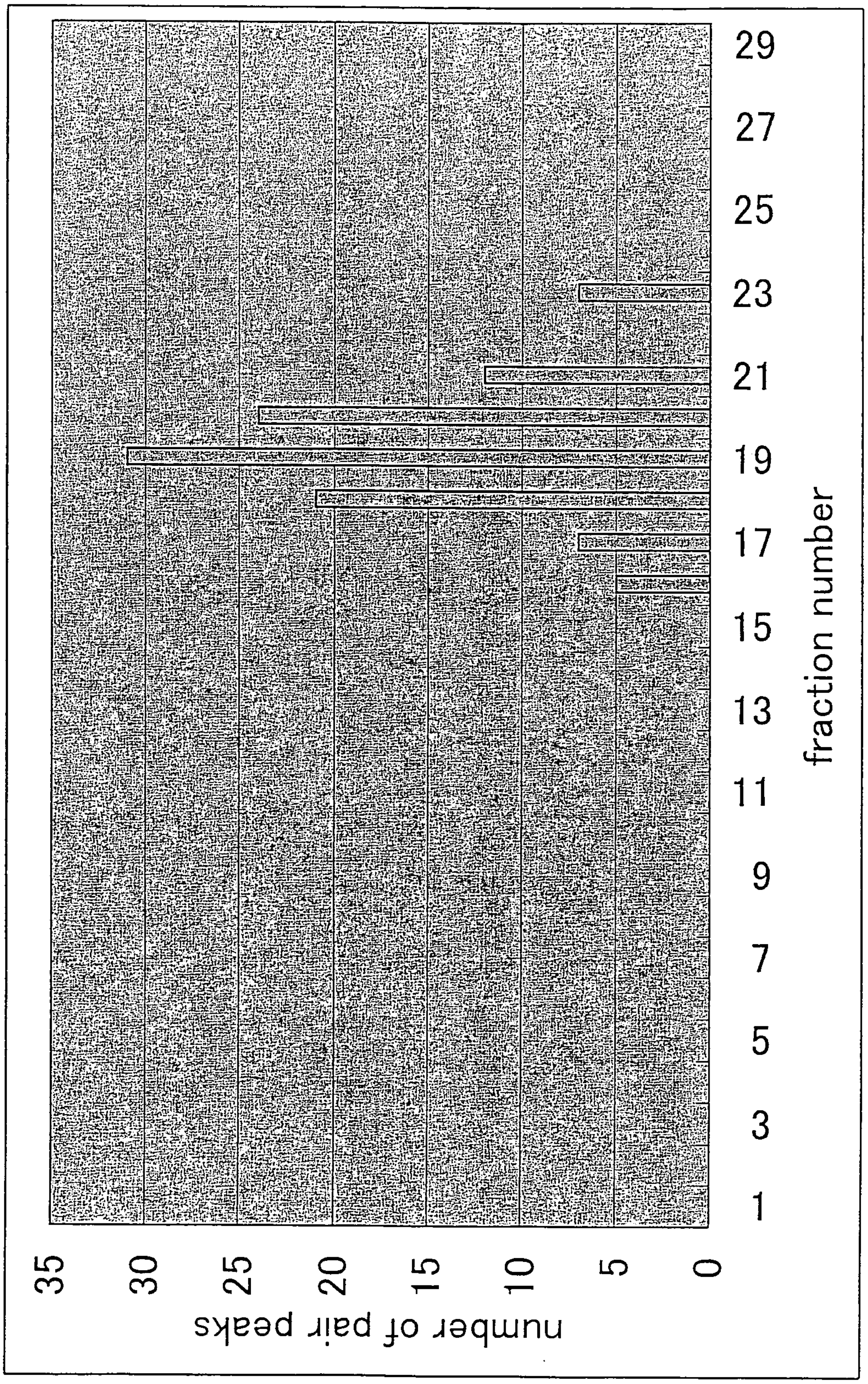
number of pair peaks
0
5
10
15
20
25
30
35
fraction number
1
3
5
7
9
11
13
15
17
19
21
23
25
27
29

Fig. 3

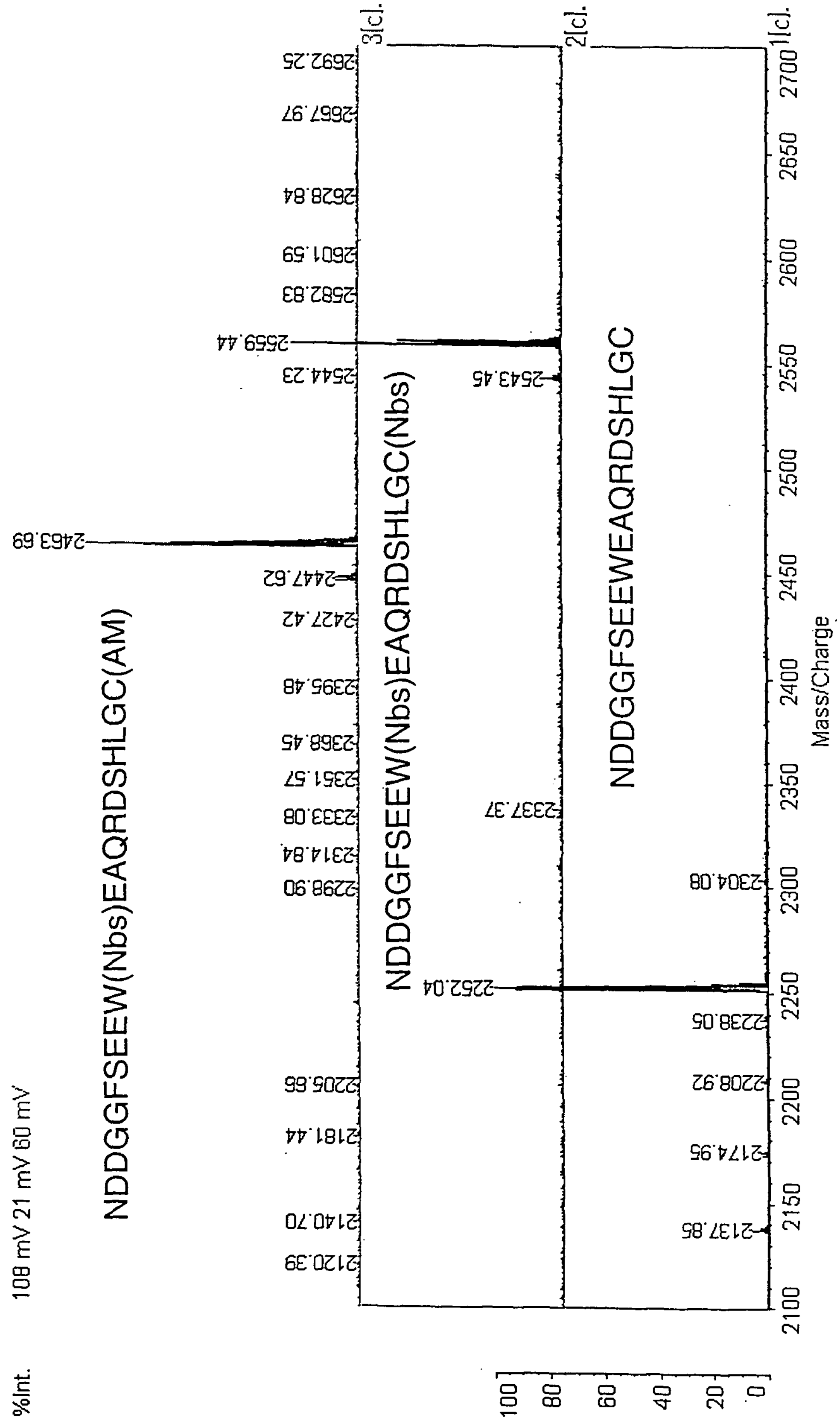
%Int.
108 mV 21 mV 60 mV
NDDGGFSEEW(Nbs)EAQRDSHLGC(AM)
NDDGGFSEEW(Nbs)EAQRDSHLGC(Nbs)
NDDGGFSEEWEAQRDSHLGC
3[c].
2[c].
1[c].
2463.69
2559.44
2543.45
2252.04
2304.08
2337.37
2120.39
2140.70
2181.44
2205.66
2298.90
2314.84
2333.08
2351.57
2368.45
2395.48
2427.42
2447.62
2544.23
2582.83
2601.59
2628.84
2667.97
2692.25
2137.85
2174.95
2208.92
2238.05
100
80
60
40
20
0
2100
2150
2200
2250
2300
2350
2400
2450
2500
2550
2600
2650
2700
Mass/Charge

Fig. 4

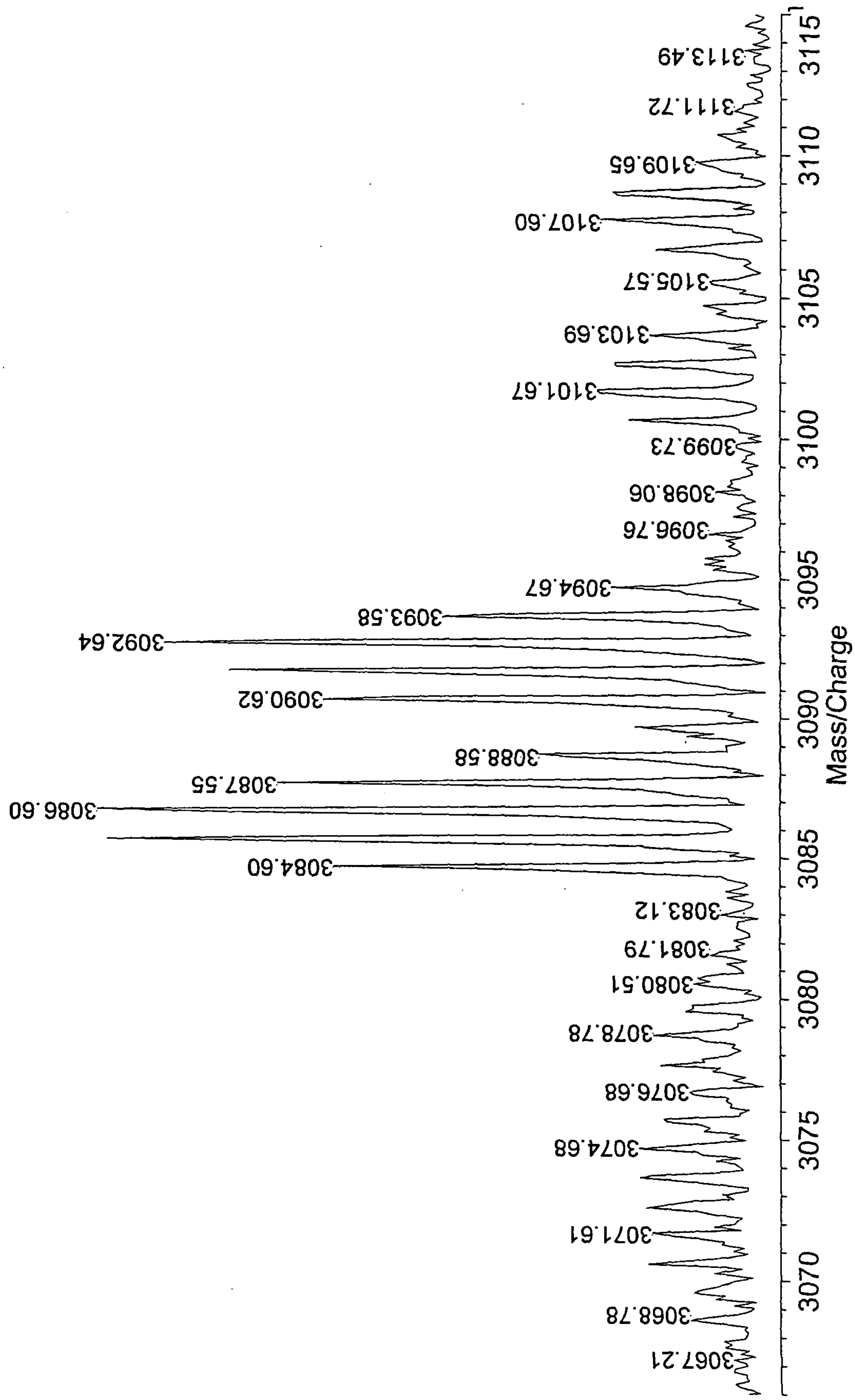
3067.21
3068.78
3071.61
3074.68
3076.68
3078.78
3080.51
3081.79
3083.12
3084.60
3086.60
3087.55
3088.58
3090.62
3092.64
3093.58
3094.67
3096.76
3098.06
3099.73
3101.67
3103.69
3105.57
3107.60
3109.65
3111.72
3113.49
3070
3075
3080
3085
3090
3095
3100
3105
3110
3115
Mass/Charge

Fig. 5

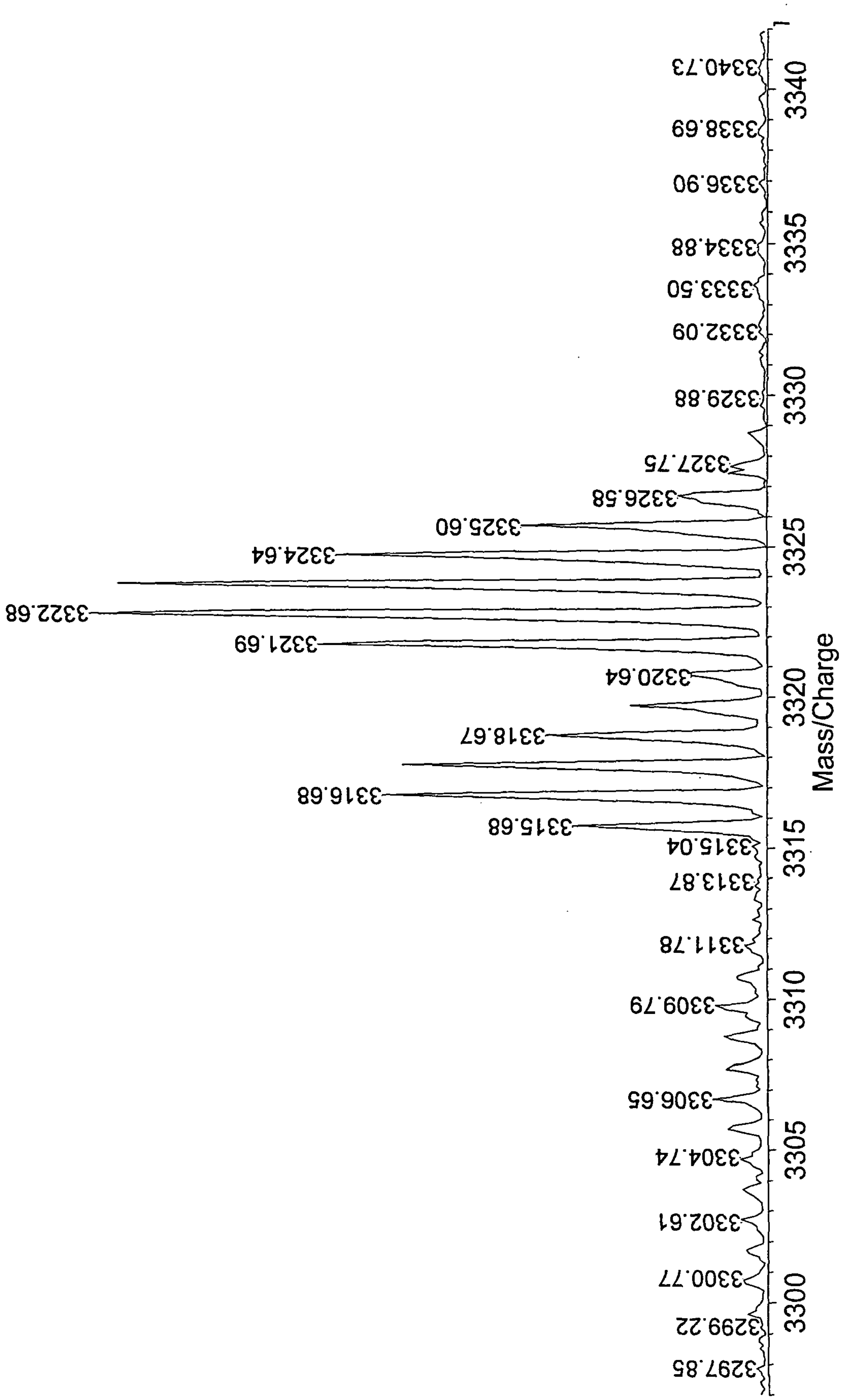
3297.85
3299.22
3300.77
3302.61
3304.74
3306.65
3309.79
3311.78
3313.87
3315.04
3315.68
3316.68
3318.67
3320.64
3321.69
3322.68
3324.64
3325.60
3326.58
3327.75
3329.88
3332.09
3333.50
3334.88
3336.90
3338.69
3340.73
3300
3305
3310
3315
3320
3325
3330
3335
3340
Mass/Charge

Fig. 6

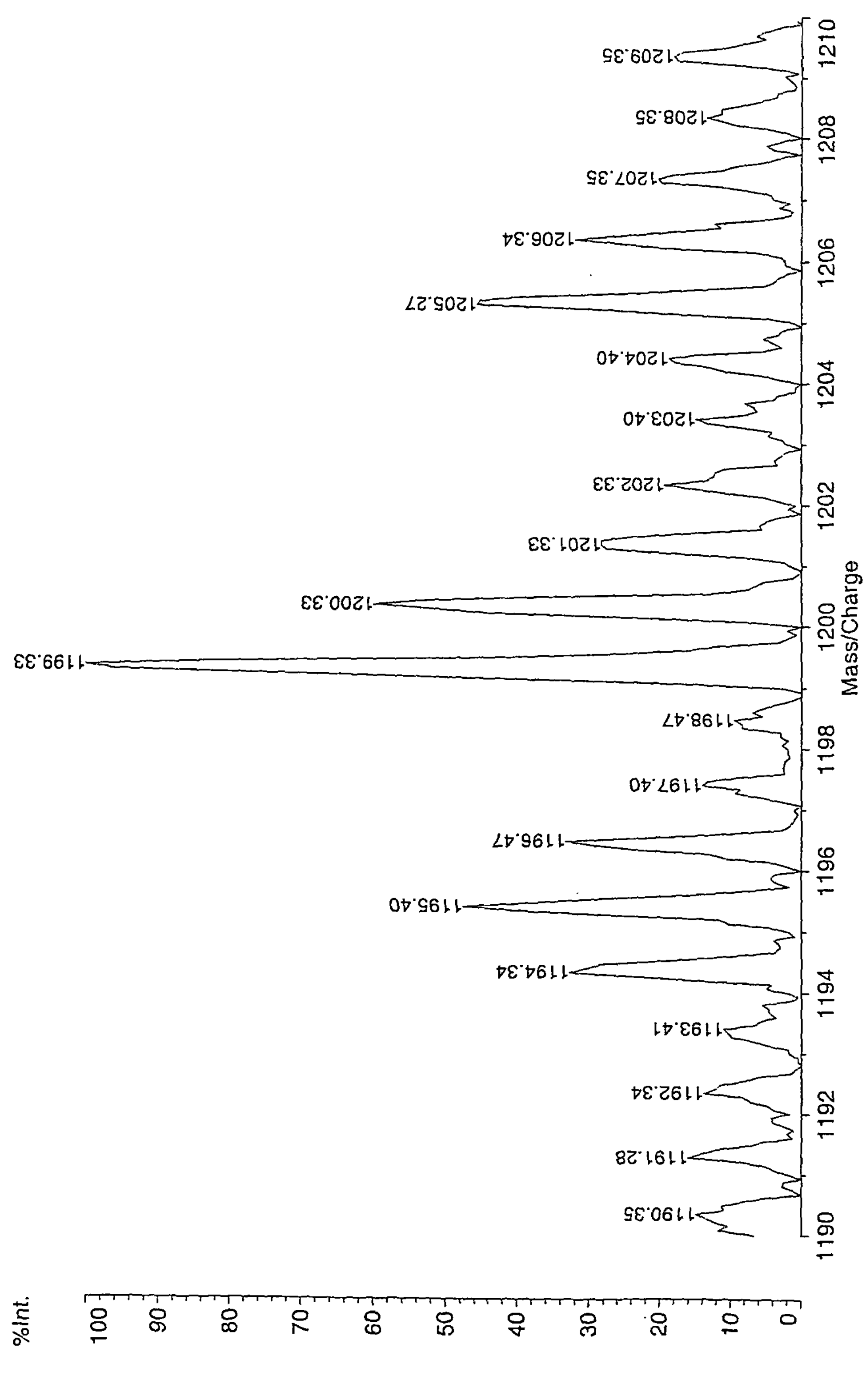
%Int.
100
90
80
70
60
50
40
30
20
10
0
1190.35
1191.28
1192.34
1193.41
1194.34
1195.40
1196.47
1197.40
1198.47
1199.33
1200.33
1201.33
1202.33
1203.40
1204.40
1205.27
1206.34
1207.35
1208.35
1209.35
1190
1192
1194
1196
1198
1200
1202
1204
1206
1208
1210
Mass/Charge

## INTERNATIONAL SEARCH REPORT

International application No.
PCT/JP03/04308

A. CLASSIFICATION OF SUBJECT MATTER
Int.Cl$^7$ C07C313/08, C07B59/00, G01N33/68, G01N27/62 , A61K38/00

According to International Patent Classification (IPC) or to both national classification and IPC

B. FIELDS SEARCHED

Minimum documentation searched (classification system followed by classification symbols)
Int.Cl$^7$ C07C313/00, C07B59/00, G01N33/00, G01N27/00, A61K38/00

Documentation searched other than minimum documentation to the extent that such documents are included in the fields searched

Electronic data base consulted during the international search (name of data base and, where practicable, search terms used)
REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| X<br>Y | MIURA, Yuzo et al., Generation, Isolation, and Characterization of N-(Arylthio)-7-tert-butyl- and N-(Arylthio)-2,7-di-tert-butyl-1-pyrenylaminyl Radicals, Journal of Organic Chemistry, 1994, Vol.59, No.12, page 3294 to 3300 | 1-9<br>1-45 |
| X<br>Y | MIURA, Yuzo et al., ESR studies of nitrogen-centered free radicals. 40. Exceptionally persistent nitrogen-centered free radicals, Journal of Organic Chemistry, 1991, Vol.56, No.23, pages 6638 to 6643 | 1-9<br>1-45 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 July, 2003 (11.07.03) | 29 July, 2003 (29.07.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/04308

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | MIURA, Yuzo et al., ESR studies of nitrogen-centered free radicals. 28. Oxygen-17-and sulfur-33-hyperfine splittings for aromatic RCO(ArS)N· radicals, Bulletin of the Chemical Society of Japan, 1986, Vol.59, No.10, pages 3291 to 3292 | 1-9<br>1-45 |
| X<br>Y | EFFENBERGER, Franz et al., Synthesis and reactions of sulfenic trifluoromethanesulfonic anhydrides, Chemische Berichte, 1982, Vol.115, No.12, pages 3719 to 3736 | 1-9<br>1-45 |
| X<br>Y | HARPP, David et al., Organic sulfur chemistry. 33. Chemistry of sulfenic sulfonic thioanhydrides, Journal of Organic Chemistry, 1979, Vol.44, No.23, pages 4135 to 4140 | 1-9<br>1-45 |
| X<br>Y | SAITO, Hiroshi et al., Matrix IR spectra and normal coordinate analysis for methanesulfenyl chloride and isotopic analogs, Spectrochimica Acta, Part A: Molecular and Biomolecular Spectroscopy, 1995, Vol.51A, No.14, pages 2447 to 2451 | 1-9<br>1-45 |
| X<br>Y | NOEL, J.P. et al., Synthesis of 3-[(2,6-14C)-2-pyridinyl-dithio]propanoic acid, a covalent coupling reagent, Journal of Labelled Compounds and Radiopharmaceuticals, 1983, Vol.20, No.11, pages 1243 to 1256 | 1-9<br>1-45 |
| Y | WO 00/11208 A1 (University of Washington), 02 March, 2000 (02.03.00), & EP 1105517 A1 & JP 2002-523058 A & US 2002/76739 A1 | 1-45 |
| Y | Edited by The Japanese Biochemical Society, "Tanpakushitsu IV Kozo Kino Sokan", Tokyo Kagaku Dojin, 1991, pages 57 to 58, 64 to 66 | 1-45 |

Form PCT/ISA/210 (continuation of second sheet) (July 1998)

SEQUENCE LISTING

<110> SHIMADZU CORPORATION

<120> Sulfenyl Compound, Labeling Reagent, and Method of Analyzing Peptide

<130> GP15-02-EP

<150> JP 2002-191496
<151> 2002-06-28

<150> JP 2002-191497
<151> 2002-06-28

<160> 15

<170> PatentIn Ver. 2.1

<210> 1
<211> 20
<212> PRT
<213> Artificial Sequence

<220>
<223> Description of Artificial Sequence: presinilin-1(331-349)-Cys

<400> 1
Asn Asp Asp Gly Gly Phe Ser Glu Glu Trp Glu Ala Gln Arg Asp Ser
1 5 10 15

His Leu Gly Cys
20

<210> 2
<211> 20
<212> PRT
<213> Artificial Sequence

<220>
<223> Description of Artificial Sequence: labelled presinilin-1(331-349)-Cys

<220>

<221> BINDING
<222> (10)
<223> 2-nitro-phenylthio-group

<220>
<221> BINDING
<222> (20)
<223> 2-nitro-phenylthio-group

<400> 2
Asn Asp Asp Gly Gly Phe Ser Glu Glu Trp Glu Ala Gln Arg Asp Ser
1 5 10 15

His Leu Gly Cys
20

<210> 3
<211> 20
<212> PRT
<213> Artificial Sequence

<220>
<223> Description of Artificial Sequence: amidemathylated labelled presinilin-1(331-349)-Cys

<220>
<221> BINDING
<222> (10)
<223> 2-nitro-phenylthio-group

<220>
<221> BINDING
<222> (20)
<223> amidemethyl-group

<400> 3
Asn Asp Asp Gly Gly Phe Ser Glu Glu Trp Glu Ala Gln Arg Asp Ser
1 5 10 15

His Leu Gly Cys
20

<210> 4
<211> 4
<212> PRT
<213> Bos taurus

<400> 4
Ile Trp Cys Lys
1

<210> 5
<211> 8
<212> PRT
<213> Bos taurus

<400> 5
Leu Asp Gln Trp Leu Cys Glu Lys
1 5

<210> 6
<211> 9
<212> PRT
<213> Bos taurus

<400> 6
Leu Asp Gln Trp Leu Cys Glu Lys Leu
1 5

<210> 7
<211> 10
<212> PRT
<213> Bos taurus

<400> 7
Val Gly Ile Asn Tyr Asn Leu Ala His Lys
1 5 10

<210> 8
<211> 5

<212> PRT
<213> Oryctolagus cuniculus

<400> 8
Gly Leu Trp Glu Lys
1 5

<210> 9
<211> 8
<212> PRT
<213> Oryctolagus cuniculus

<400> 9
Gly Leu Trp Glu Lys Ala Phe Lys
1 5

<210> 10
<211> 14
<212> PRT
<213> Oryctolagus cuniculus

<400> 10
Leu Ile Ser Trp Tyr Asp Asn Glu Phe Gly Tyr Ser Asn Arg
1 5 10

<210> 11
<211> 4
<212> PRT
<213> Gallus gallus

<400> 11
Glu Trp Thr Arg
1

<210> 12
<211> 8
<212> PRT
<213> Gallus gallus

<400> 12
Glu Trp Thr Arg Met Val Ile Arg
1 5

<210> 13
<211> 6
<212> PRT
<213> Rattus norvegicus

<400> 13
Ala Trp Ala Val Ala Arg
1 5

<210> 14
<211> 7
<212> PRT
<213> Rattus norvegicus

<400> 14
Asn Thr Ala Ala Trp Ala Lys
1 5

<210> 15
<211> 5
<212> PRT
<213> Rattus norvegicus

<400> 15
Trp Lys Ile Arg Lys
1 5